(19) Europäisches Patentamt
European Patent Office
Office européen des brevets

(11) Numéro de publication: **0 035 949**
**B1**

# (12) FASCICULE DE BREVET EUROPEEN

(45) Date de publication du fascicule du brevet: **19.09.84**

(51) Int. Cl.$^3$: **B 01 D 39/04**

(21) Numéro de dépôt: **81400379.4**

(22) Date de dépôt: **11.03.81**

## (54) Filtre textile pour flux gazeux, comprenant des fibres organiques greffées avec des monomères actifs.

(30) Priorité: **12.03.80 FR 8005531**

(43) Date de publication de la demande:
**16.09.81 Bulletin 81/37**

(45) Mention de la délivrance du brevet:
**19.09.84 Bulletin 84/38**

(84) Etats contractants désignés:
**AT BE DE GB IT LU NL SE**

(56) Documents cités:
**DE-A-2 737 070**
**FR-A-2 155 661**

**TAPPI, vol. 55, no. 6, juin 1972, Atlanta, US R.B. PHILLIPS et al.: "Modification of Pulp and Paper by Graft Copolymerization", pages 858-867**
**TAPPI, vol. 45, no. 5, mai 1962 Atlanta, US E. SCHWAB et al.: "Paper Grafted with Vinyl Monomers Using the Ceric Ion Method", pages 390-400**

(73) Titulaire: **DOLLFUS & NOACK**
**F-68390 Sausheim (FR)**

(72) Inventeur: **Dri, Jean-Pierre**
**18, Rue de Soultz Uffholtz**
**F-68700 Cernay (FR)**

(74) Mandataire: **Debay, Yves et al**
**NOVAPAT-CABINET CHEREAU 107, boulevard Péreire**
**F-75017 Paris (FR)**

(56) Documents cités:
**JOURNAL OF POLYMER SCIENCE, Part C, no. 4, 1963 New York, US G.N. RICHARDS et al.: "Graft Polymerization on Cellulosic Materials, Part I. Cation-Exchange Membranes from Paper and Acrylic Acid" pages 1251-1260**
**JOURNAL OF POLYMER SCIENCE: Part C, no. 4, 1963 New York, US I. SAKURADA et al.: "Radiation-Induced Graft Copolymerization onto Cellulose and Polyvinyl Alcohol Fibers with Binary Mixtures of Comonomers", pages 1233-1249**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**EP 0 035 949 B1**

Courier Press, Leamington Spa, England.

## Description

La présente invention concerne la filtration et l'épuration de gaz, et plus particulièrement, les filtres à effets de rétention physique et chimique.

En matière de filtration gazeuse, les filtres à effet mécanique sont très largement répandus, les qualités du filtre se définissant essentiellement par les dimensions des pores réalisés par les parois et le substrat filtrants. Les performances de ces filtres peuvent être améliorées en recourant aux techniques dites des filtres humides avec les inconvénients afférents de conditionnement et de tenue dans le temps.

Il est connu par le brevet allemand DE—A—2 737 070, un procédé pour purifier les gaz contenant des acides minéraux, au moyen d'une structure fibreuse comprenant des substances absorbeuses par un phénomène d'échange d'ions. Ce procédé est applicable sur des produits minéraux qui se dissocient chimiquement dans l'eau en ions pour être recyclés par régénération de la résine.

Pour la rétention non seulement mécanique mais également chimique de particules indésirables dans un courant gazeux, on a également proposé des filtres composites utilisant des substances actives confinées qui, même dans le cas de filtres secs, causent des problèmes notables de rétention du produit actif qui ne sont en général résolus qu'en optimisant les parois de rétention, au prix toutefois de pertes de charge considérables et souvent rédhibitoires.

Parmi ces produits actifs, on sait également que nombre de monomères présentent, à des titres divers, pour certaines classes de particules nuisibles, des caractéristiques d'affinité et de rétention chimiques qui peuvent présenter un intérêt dans les techniques du filtrage, avec toutefois les inconvénients susmentionnés de contention et de rétention de ces matériaux actifs.

On sait également, dans un autre domaine, que des fibres, et plus particulièrement les fibres textiles, peuvent être greffées avec certains monomères organiques pour leur conférer des propriétés nouvelles notamment en ce qui concerne l'affinité tinctoriale et l'attribution de caractéristiques hydrophiles ou d'ininflammabilité.

Ainsi, il est connu par l'article du TAPPI, vol 55 n° 6, juin 1972, pages 858 à 867, Atlanta US, publié au nom de PHILLIPS R.B. & al et intitulé "Modification of Pulp and Paper by Graft Copolymerisation", de greffer des monomères sur des filtres en papier pour en améliorer les caractéristiques physiques uniquement et plus particulièrement pour augmenter leur tenue mécanique.

La présente invention a pour objet de proposer une nouvelle classe de filtres secs à substrat filtrant fibreux présentant des qualités de rétention et de fixation fortement améliorées et facilement modulables, sans présenter de risques d'entraînement notables tout en offrant une durée de vie largement améliorée.

La présente invention a pour autre objet de proposer des filtres de configuration simple, de faible coût de fabrication, combinant de façon optimale et avec une grande versatilité, des qualités de rétention mécanique améliorées et de rétention chimique pour le filtrage et le traitement de flux gazeux.

Pour ce faire, selon une caractéristique de la présente invention, un tel filtre sec, pour filtrer mécaniquement et purifier chimiquement un flux gazeux véhiculant des particules à éliminer, comprenant un substrat filtrant, constitué d'au moins une nappe fibreuse comportant des fibres organiques, naturelles ou synthétiques, au moins une partie des fibres du substrat filtrant étant greffé avec au moins un monomère organique, capable de retenir physiquement et chimiquement au moins certaines des particules véhiculées dans le flux gazeux, et caractérisé en ce qu'au moins une partie des fibres est greffée avec des composés choisis parmi les groupes comprenant les peracides, les mercaptants, les éthylacétoacétates et les éthylacétamendomalonates.

Conformément à la présente invention, le greffage sur des fibres d'un substrat filtrant de monomères réactifs ayant une affinité pour certaines classes de particules solides ou liquides véhiculées dans un flux gazeux permet ainsi de combiner, de façon simple et particulièrement efficace, les avantages propres aux agents greffés en tant qu'agents réactifs, et aux caractères de filtration mécanique du tapis ou lit de fibres ainsi greffées, en permettant de répartir au mieux les agents chimiques au sein du substrat filtrant sans les risques usuels d'entraînement non seulement des particules retenues mais également de l'agent actif et/ou des particules fixées par ce dernier, tout en autorisant un agencement de filtrage mécanique optimisé au prix de pertes de charges contrôlables à un faible niveau.

D'autres avantages de la présente invention ressortiront de la description suivante de modes de réalisation, donnés à titre illustratif mais nullement limitatif.

Différentes techniques de greffage ont été développées ces dernières années, notamment dans le but de modifier les caractéristiques physiques des éléments à greffer, notamment des tissus textiles, ces techniques de greffage pouvant se classer selon trois grands groupes:

— le greffage ionique par voie humide utilisant des amorceurs tels que les ions cériques, parmanganates, ferriques, utilisés en milieu acide minéral. Dans le cas plus particulier des fibres, notamment de cellulose ou de ses dérivés, celles-ci sont imprégnées de solutions acides d'amorceur puis traitées par des monomères choisis dans des conditions spécifiques à chaque produit, en phase vapeur ou azéotropique pour les dérivés acryliques, ou en phase

solvant. Le greffage de sels d'ammonium quaternaires s'effectue par contre sans amorceur en présence d'une solution de base forte, comme notamment décrit dans le brevet français FR—A—1.585.665. Dans tous les cas, le tissue est lavé, rincé après réaction puis séché. Pour le greffage en phase solvant, on favorise l'apparition de carbo-cations sur des produits comportant une double liaison en présence notamment de $AlCl_3$ ou $ZnCl_2$. Les carbo-cations se fixent sur les matières en suspension dans le solvant, par exemple la silice, le noir de carbone ou les oxydes d'aluminium.

— Le greffage à l'aide de rayonnement U.V. Ce rayonnement étant de faible énergie, il est nécessaire d'utiliser des photo-initiateurs de réaction qui peuvent être des groupes carbonyle, la benzophénone ou les vapeurs de diacétyle. Cette méthode présente l'avantage de permettre de modifier des fibres réputées inertes telles que le polypropylène ou le polyester.

— Le greffage à l'aide de particules accélérées à haute énergie ou de rayons gamma fournis par exemple par une source radio-active de cobalt 60. Sous l'action des particules à haute énergie, il se forme au sein de la fibre organique des radicaux libres par arrachage d'atomes d'hydrogène, ces radicaux instables favorisant l'amorçage du greffage des monomères choisis. Ainsi, si F—CH—F représente symboliquement la fibre et M le monomère de greffon, en fin de réaction on obtiendra une fibre greffée

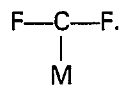

La réaction s'effectue soit en irradiant la fibre seule en présence ou non d'humidité, soit en irradiant la fibre imprégnée de monomères.

Le greffage à l'aide de particules à haute énergie sera de préférence utilisé pour la réalisation des filtres selon la présente invention, comme on le verra ci-dessous, car, par un processus radicalaire, il est possible de greffer deux ou même plusieurs monomères différents sur une même fibre, quelle que soit sa nature, chaque homopolymère greffé conservant ses propriétés spécifiques d'agent réactif. De cette manière, pour élargir le champ d'application du filtre, il est possible d'apporter plusieurs propriétés différentes à une même fibre. De plus, l'utilisation industrielle d'une telle réaction radicalaire fait appel à une dépense moindre d'énergie comparativement aux autres procédés, la réaction se faisant à température ambiante, donc sans la nécessité d'apport calorifique. La réaction peut s'effectuer en amont, sur les fibres brutes, mais également sur les fibres tissées ou aiguilletées; pour un tissu sec, les opérations préliminaires de trempage et foulardage sont ainsi supprimées.

Dans un premier mode de réalisation, on décrira un filtre selon la présente invention conçu pour la rétention des odeurs et des graisses dans les hottes ménagères.

La préparation des fibres pour l'obtention d'un support textile formant au moins une partie du substrat filtrant s'effectue par l'action d'un flux de particules accélérées. Comme précédemment évoqué, cette opération peut avoir lieu sur un voile de fibres ou sur le produit déjà préalablement aiguilleté. L'irradiation est instantanée, et n'est fonction que de la puissance de l'accélérateur de particules. Une puissance généralement admise pour ce type d'irradiation est comprise entre 20 et 50 kW, permettant une irradiation de 500 KeV à plus d'un 1 MeV, une puissance de 600 KeV étant généralement suffisante. L'augmentation du taux d'irradiation, et donc l'augmentation du nombre de sites réactifs, peut être obtenue en faisant passer plusieurs fois le matériau fibreux sous le flux énergétique, par exemple en utilisant un système de passage dit en porte-feuille

Le substrat filtrant de départ, constitué d'un assemblage lâche de fibres organiques, naturelles ou synthétiques, sous forme péroxydée ou non, est mis en contact pour greffages successifs avec les familles de monomères suivants:

(a) monomères chlorés ou sels d'ammonium, ou de préférence des phosphonates d'alkyle ayant en bout de chaîne un groupement vinylique, notamment. Ces produits confèrent aux fibres ainsi traitées un pouvoir ignifugeant, un taux de greffage des fibres de l'ordre de 20 % suffisant par exemple à rendre celles-ci auto-extinguibles.

(b) monomères d'acrylates d'alkyle comportant 4,5 ou 6 atomes de carbone, ou acrylonitrile, ou isoprène, ou copolymères butadiène-styrène, notamment. Ces composés présentent une affinité marquée aux graisses, l'absorption de ces dernières se faisant, dans un premier temps, sous forme de solution graisse-polymère, puis, dans une second temps, en effectuant une réaction, chimique de pontage. Conformément à la présente invention, l'élément de substrat ainsi réalisé empêche les graisses retenues, plus exactement fixées, d'être réentraînées hors du filtre par la circulation d'air chargé de vapeur d'eau, contrairement aux filtres actifs existants;

(c) acrylonitrile, ou mercaptans, ou péracides, ou éthylacétoacétates, éthylacétamidomalonates, et les dérivés aminés. Ces composés confèrent aux fibres de substrat filtrant une réactivité vis-à-vis des aldéhydes ou des dérivés insaturés, ce qui permet en particulier de fixer chimiquement l'acroléine résultant de la décomposition des graisses.

On comprendra qu'un tel filtre selon la présente invention peut être également obtenu, en variante, en traitant plusieurs nappes de fibres séparément avec les composés (a) à (c) et en ne les réunissant, les tissant ou les aiguilletant qu'après copolymérisation.

Les filtres habituels, retiennent les graisses

par dépôt de celles-ci sur le substrat filtrant et ne peuvent en aucun cas retenir les produits plus volatils comme l'acroléine. De plus, comme précédemment évoqué, dans le cas de simple dépôt, les graisses sont très rapidement réentraînées par l'air saturé de vapeur d'eau dans les conduites d'évacuation où elles se redéposent et constituent ainsi un danger de propagation d'incendie.

An contraire, le filtre selon la présente invention, non seulement retient les graisses et les produits volatiles, mais également les fixe de façon efficace et durable. Un substrat fibreux ayant un poids au mètre carré compris entre 150 et 500 grammes — typiquement 300 grammes par par mètre carré — et ayant une perméabilité de l'air sous 20 mm d'eau comprise entre 4000 et 15000 $m^3/m^2$/heure — de préférence 8 à 10000 $m^2/m^3$/heure, permet de retenir, par $m^2$:

— plus de 100 grammes de graisses sous forme liée;

— plus de 40 grammes d'hydrocarbures lourds dus à la combustion et à la décomposition de graisses; et

— plus de 90 % de l'acroléine formée, dont 50 % au moins sous forme liée, pour une teneur en acroléine dans l'air à filter inférieure à 0,05 $mg/m^3$;

ce que correspond en moyenne aux effluents émis dans une cuisine ménagère pendant 2 à 3 mois pour une famille de 3 personnes.

Dans un second mode de réalisation, on décrira un support textile utilisé pour filtrer et débarrasser l'air d'éléments indésirables avant de l'introduire dans un local ou un atelier.

Ce mode de réalisation s'applique aussi bien à l'aspiration et/ou l'introduction dans l'enceinte d'une usine, d'air débarrassé des odeurs, des poussières et des produits organiques, qu'à une épuration de l'air aux postes de travail, notamment dans les industries des peintures et vernis.

Conformément à la présente invention, il est possible de choisir judicieusement les monomères de greffage pour conférer au substrat filtrant une affinité particulière vis-à-vis de produits spécifiques à éliminer, par exemple des dérivés insaturés, les aldéhydes (comme précédemment évoqué) ou les esters responsables des odeurs en greffant au moins une partie des fibres du substrat filtrant avec des composés tels que les péracides, les mercaptans ou l'acrylonitrile, comme dans l'exemple 1. Par ailleurs, le greffage, sur au moins une partie des fibres du substrat filtrant, de dérivés aminés permet en outre de retenir chimiquement les vapeurs acides faibles sous forme de sels jusqu'à 50 % en poids du filtre pour un taux de greffage de 20 % des fibres greffées contenus dans le substrat filtrant. Le greffage avec des dérivés sulfonés ou non de polyacides acryliques permet de retenir d'autre par des vapeurs basiques jusqu'à 100 % en poids de fibres greffées au filtre. On peut en outre introduire dans le substrat filtrant des fibres organiques, naturelles ou synthétiques, greffées de butadiène-styrène typiquement avec un taux de greffage de 20 %, ce qui ainsi permet au filtre de retenir jusqu'à 200 % de son propre poids en fibres greffées, de benzène, toluène, hexane ou acétone.

Dans un troisième mode de réalisation, on décrira un filtre selon la présente invention pour l'assainissement de l'air pollué par la fumée de tabac et convenant tout particulièrement pour les locaux publics, les transports en commun, les avions ou encore les salles de conférence et les salles d'attente.

Les dispositifs de filtrage actuels sont basés sur le principe de la rétention des particules solides et des goudrons au moyen d'un filtre mécanique généralement associé à un électrofiltre. Ces dispositifs, comme mentionnés dans la partie introductive de la présente demande, ne permettant pas une rétention efficace des particules visibles, du fait que celle-ci se trouvent simplement déposées sur les substrats filtrants, de sorte que des composés comme l'acide cyanhydrique, le monoxyde de carbone, les goudrons, la nicotine ou encore l'acroléine, ne sont pas retenus, seul l'effet de dilution des polluants dans le local permettant d'atteindre un assainissement tout à fait limité.

Conformément à la présente invention, un substrat filtrant est préparé en irradiant des fibres organiques sous un flux de particules accélérées, avec une puissance de 30 kW environ, de façon que les particules aient une énergie de 600 KeV, pour un temps d'irradiation variant de 0,1 à quelques secondes selon le pourcentage de radicaux libres désirables, un temps d'irradiation d'une seconde étant suffisant pour obtenir le taux de greffage souhaité de l'ordre de 20 %. Avec ce processus d'irradiation, une partie des fibres ainsi irradiées, destinées à constituer le substrat filtrant, est ensuite mise en contact avec des sels minéraux d'époxypropylammonium ou d'époxypropyltriméthylammonium où 1, 2 ou même 3 atomes d'hydrogène peuvent être remplacés par des radicaux méthyle, éthyle ou propyle. Des molécules insaturées aminées peuvent également être utilisées à la place des groupes ammonium comme décrit dans l'exemple 1. Les molécules n'ayant pas réagi sont éliminées par lavage au solvant ou par un mélange eau-solvant en présence d'une base forte (par exemple soude à une concentration normale de 0,1 á 3N).

Une seconde partie des fibres organiques irradiées est mise en contact avec des vapeurs ou des solutions solvant d'acide acrylique en présence d'acide oxalique. L'excès d'homopolymère formé est éliminé par lavage au moyen d'un mélange eau-solvant organique (alcool, benzène ou hexane).

Une troisième partie des fibres irradiées est enfin greffée de la même manière en présence d'acrylonitrile ou de dérivés insaturés porteurs d'un groupement SH ayant un hydrogène labile, comme décrit dans le premier exemple.

Le substrat filtrant est ensuite obtenu en opérant un mélange intime de ces trois catégories de fibres greffées par un moyen communément utilisé, par exemple par cardage, avant de réaliser l'aiguilletage ou le tissage de ces fibres pour constituer un substrat filtrant sensiblement homogène.

Le substrat filtrant ainsi obtenu permet de retenir de façon durable:

— la nicotine;

— 100 % de l'acide cyanhydrique;

— plus de 90 % de l'acroléine, pour une teneur en acroléine inférieure à 0,05 mg/m$^3$ dans l'air à filtrer; et

— plus de 50 % du monoxyde de carbone, l'air rejeté après filtration ayant une teneur en CO inférieure à 10 ppm.

Ces valeurs sont valables pour le traitement de la fumée de 5000 cigarettes environ pour un substrat filtrant en feutre de 600 grammes ayant une surface unitaire de 1 m$^2$. Un tel substrat filtrant peut par exemple, être utilisé comme cartouche filtrante dans un appareil qui peut être constitué d'une partie cylindrique d'un diamètre de 30 cm environ pour une hauteur d'environ 30 cm, ce cylindre étant muni, à ses parties inférieure et supérieure d'ouvertures favorisant le passage d'air. Le passage d'air forcé à travers le substrat se fait de préférence au moyen d'un petit ventilateur électrique de faible puissance, de l'ordre de 50 à 100 Watts.

Dans un quatrième mode de réalisation, on décrira un substrat filtrant plus particulièrement destiné à des utilisations dans les hôpitaux et les collectivités.

Il est bien connu qu'une des causes non négligeable de propagation d'épidémies est la transmission de germes par le sol, mais surtout par l'air. Un moyen d'atténuer ces risques consiste, au stade de l'admission d'air dans des locaux, de le traiter à l'aide de composés tels que par exemple des sels organiques de cuivre, d'argent ou de métaux lourds, certains peroxydes tels que le peroxyde de benzoyle, des acides à forte adhérence vis-à-vis des matières protiniques telles que les polyacides acryliques, ainsi que les sels d'hydroxypropylammonium. On notera ici, que les virus, eux, ne peuvent être efficacement affectés, que par le chlore ou l'iode.

Pour la préparation du substrat filtrant selon la présente invention, des fibres organiques, par exemple sous forme de voile, sont tout d'abord irradiées comme dans l'exemple précédent, en présence d'air et d'humidité, ce qui provoque la formation de peroxydes stables à température ambiante. La masse de fibres irradiées est ensuite séparée en deux parties pour des greffages distincts:

Une première partie est traitée par du chlorure d'époxypropylammonium ou de l'iodure d'époxypropyltriméthylammonium à une température supérieure à 50°C, l'homopolymère formé n'étant pas extrait.

La deuxième partie est traitée par un mélange d'acrylonitrile et d'acrylate d'argent ou d'acides acryliques et d'acrylates d'argent à une température par exemple supérieure à 50°C, l'homopolymère formé étant extrait à l'eau.

Les deux parties greffées avec des monomères différents sont intimement mélangées et aiguilletées pour former une masse filtrante homogène. Il va de soi qu'un tel substrat filtrant peut être obtenu par la juxtaposition de lits de fibres préalablement irradiées après aiguilletage, soumis, l'un à la première réaction, l'autre à la seconde.

Le substrat filtrant ainsi obtenu réalise bien évidemment, selon l'aménagement interne de ce substrat, le dépoussiérage mécanique de l'air, mais procure en outre une action sanitisatrice marquée en ce sens qu'il limite la prolifération des germes, les inhibe et les détruit. Comme pour les autres filtres selon la présente invention, le greffage fixant les monomères sur les fibres constitutives du substrat filtrant par une véritable liaison chimique empêche que ces monomères et leurs mélanges avec différents germes ne soient ré-entraînés par l'air à épurer, contrairement à ce qui risquerait de se passer si des agents actifs similaires étaient simplement déposés sur une masse filtrante.

Les composés tels que les sels d'ammonium quaternaires ont des effets bactériostatiques très significatifs, leur mode d'action étant fondé sur la dépolymérisation des protéines, ce qui concourt ainsi à la destruction des parois des cellules bactériennes. De même, les aldéhydes réagissent fortement sur les groupes aminés de la paroi cellulaire des bactéries. Les pseudomonas, les microbactéries, et les spores sont, quant à eux, fortement affectés par les aldéhydes, les phénols, les sels d'ammonium quaternaires et les sels de métaux lourds.

L'utilisation d'un tel filtre n'a pas pour but affiché de stériliser totalement l'air ambiant, quels que soient les germes présents, mais vise à réaliser une sanitisation efficace de cet air avant son introduction dans les locaux ou avant rejet dans l'atmosphère.

On notera, à titre incident, que le substrat filtrant ainsi obtenu peut être utilisé avec profit pour le nettoyage des sols, en évitant ainsi que la présence de poussières absorbées par les masses humides de nettoyage favorisent la prolifération de germes in situ.

La présente invention n'est pas limitée aux exemples décrits ci-dessus dans leur application préférentielle et sont susceptibles de modifications et de variantes qui apparaîtront à l'homme de l'art. Ainsi, dans le domaine du dépoussiérage mécanique en général, une attention toute particulière doit être portée sur les média filtrants selon la présente invention, dont les composés greffés peuvent reticuler et réaliser ainsi une matière filtrante tridimensionnelle stable, notamment pour les substrats filtrants réalisés en non tissés. Les procédés actuels font appel à des résines en latex ou en solution dans des solvants, qui nécessitent d'importantes dépenses d'énergie pour évacuer les solvants et effectuer la polymérisation. Au contraire, dans les substrats selon la présente invention, le greffage par irradiation se fait à sec et, selon le monomère choisi, il n'y a pas lieu généralement d'éliminer l'homopolymère formé comme évoqué notamment en relation avec la première partie de fibres traitée dans le mode de réalisation décrit en dernier ci-dessus.

Par ailleurs, au niveau filtration purement mécanique, il est possible de greffer sur les fibres des molécules permettant de diminuer les interstices existant dans le réseau constitué par le substrat filtrant, par exemple en dosant les pourcentages d'homopolymères formés, ces taux étant ajustables de 1 à 200 % en poids par rapport aux fibres à traiter. On peut ainsi accéder à une nouvelle dimension en filtration sèche procurant, outre les caractéristiques de fixation chimique déjà évoquées, des caractéristiques de filtration purement mécanique très largement améliorées. Ainsi, un filtre fibreux non traité, retenant de 98 à 99 % de poussières de silice normalisée comprenant 2 % de poussières inférieures à 1 micron, peut retenir, après greffage, jusqu'à plus de 99, 95 % ces poussières.

Un autre aspect important, tenant à la filtration purement mécanique, permis par le greffage des fibres, consiste en le traitement anticolmatage des substrats filtrants. On sait que, pour faciliter le battage mécanique débarrassant un substrat filtrant des particules y adhérant et éviter ainsi le colmatage, on peut utiliser notamment des silicones appliqués sur le substrat filtrant. L'expérience a toutefois montré que, du fait du simple dépôt de ces silicones, sur les matériaux du substrat filtrant, l'élimination des particules adhérant au filtre entraîne, à la longue, également l'élimination du revêtement de silicones, aboutissant ainsi à un colmatage très rapide du filtre. Conformément à la présente invention, le substrat filtrant peut être rendu anti-adhérant de façon permanente en greffant au moins les fibres constituant la couche superficielle du substrat filtrant avec des monomères de dérivés perfluorés, par exemple le 2.2.2-trifluorométhylméthacrylate d'ammonium en milieu diméthylformamide ou le monochlorotrifluoroéthylène en présence de peroxydes ou de silanes halogénés tels que le diméthyldichlorosilane. De par le mode de fixation par greffage, ce traitement résiste à l'usure, ce qui n'est pas le cas avec des produits simplement déposés comme préalablement évoqué. Un greffage de l'ordre de 10 % est en outre suffisant pour rendre de façon permanente, hydrophobes et anti-adhérantes les fibres usuelles.

## Revendications

1. Filtre sec, pour filtrer mécaniquement et purifier chimiquement un flux gazeux véhiculant des particules à éliminer, comprenant un substrat filtrant constitué d'au moins une nappe fibreuse comportant des fibres organiques, naturelles ou synthétiques, au moins une partie des fibres du substrat filtrant étant greffée avec au moins un monomère organique capable de retenir physiquement et chimiquement au moins certaines des particules véhiculées dans le flux gazeux, caractérisé en ce qu'au moins une partie des fibres est greffée avec des composés choisis parmi les groupe comprenant les péracides, les mercaptans, les éthylacétoacétates et les éthylacétamidomalonates.

2. Filtre selon la revendication 1, caractérisé en ce qu'au moins une partie des fibres est greffée avec des derivés d'ammonium quaternaires.

3. Filtre selon la revendication 2, caractérisé en ce qu'au moins une partie des fibres est greffée avec du chlorure d'epoxypropylammonium ou de l'iodure d'epoxypropyltriméthylammonium et leurs dérivés.

4. Filtre selon l'une quelconque des revendications précédentes, caractérisé en ce qu'au moins une partie des fibres est greffée avec des monomères choisis parmi le groupe comprenant l'acide acrylique, les monomères d'acrylate d'alkyles comportant 4, 5 ou 6 atomes de carbone et l'acrylonitrile.

5. Filtre selon l'une quelconque des revendications précédentes, caractérisé en ce qu'au moins une partie des fibres est greffée avec des dérivés aminés.

6. Filtre se on l'une quelconque des revendications précédentes, caractérisé en ce qu'au moins une partie des fibres est greffée avec des composés choisis parmi les monomères comprenant des doubles liaisons, l'isoprène, les copolymères butadiène-styrène et polydiènes.

7. Filtre selon l'une quelconque des revendications précédentes, caractérisé en ce qu'au moins une partie des fibres est greffée avec des silanes ou des monomères fluorés.

8. Filtre selon l'une quelconque des revendications précédentes, caractérisé en ce que, pour chaque fibre greffés, le taux de greffage est supérieur ou égal à 20 %.

## Patentansprüche

1. Trockenfilter zum mechanischen Filtrieren und chemischen Reinigen eines zu entfernende Teilchen mitführenden Gasstromes, mit einem Filtersubstrat, das aus wenigstens einer Faserlage besteht, die organische, natürliche oder synthetische Fasern aufweist, wobei wenigstens ein Teil der Fasern des Filtersubstrats mit wenigstens einem organischen Monomer gepfropft ist, das in der Lage ist, wenigstens einige der im Gasstrom miggeführten Teilchen physikalisch und chemisch zurückzuhalten, dadurch gekennzeichnet, daß wenigstens ein Teil der Fasern mit Zusammensetzungen gepfropft ist, die ausgewählt sind aus der Gruppe umfassend die Persäuren, die Mercaptane, die Ethylacetoacetate und die Ethylacetamidomalonate.

2. Filter nach Anspruch 1, dadurch gekennzeichnet, daß wenigstens ein Teil der Fasern mit

quaternären Ammonium-Derivaten gepfropft ist.

3. Filter nach Anspruch 2, dadurch gekennzeichnet, daß wenigstens ein Teil der Fasern mit Epoxypropylammoniumchlorid oder Epoxypropyltrimethylammoniumiodid und ihren Derivaten gepfropft ist.

4. Filter nach einem der vorhergehenden Ansprüche, dadurch gekennzeichnet, daß wenigstens ein Teil der Fasern mit Monomeren gepfropft ist, die ausgewählt sind aus der Gruppe umfassend die Acrylsäure, die Monomere des Alkylacrylate, enthaltend 4, 5 oder 6 Kohlenstoffatome und das Acrylnitril.

5. Filter nach einem der vorhergehenden Ansprüche, dadurch gekennzeichnet, daß wenigstens ein Teil der Fasern mit Amino-Verbindungen gepfropft ist.

6. Filter nach einem der vorhergehenden Ansprüche, dadurch gekennzeichnet, daß wenigstens ein Teil der Fasern gepfropft ist mit Zusammensetzungen, die ausgewählt sind aus den Monomeren die Doppelbindungen aufweisen, dem Isopren, den Copolymeren Butadien-Styrol und Polydienen.

7. Filter nach einem der vorhergehenden Ansprüche, dadurch gekennzeichnet, daß wenigstens ein Teil der Fasern mit Silanen oder fluorierten Monomeren gepfropft ist.

8. Filter nach einem der vorhergehenden Ansprüche, dadurch gekennzeichnet, daß für jede gepfropfte Faser der Pfropfungsgrad größer oder gleich 20 % beträgt.

**Claims**

1. A dry filter, for mechanically filtering and chemically purifying a gaseous flux carrying particles to be eliminated, which comprises a filtering substrate including at least a fibrous mat formed of natural or synthetic organic fibers, at least a portion of the fibers of said filtering substrate being grafted with at least one organic monomer capable of physically and chemically retaining at least some of said particles carried in said gaseous flux, wherein a portion of said fibers is grafted with compounds chosen in the groups comprising peracids, mercaptans, ethylacetoacetates and ethylacetamidomalonates.

2. The filter according to claim 1, wherein at least a portion of said fibers is grafted with quaternary ammonium derivatives.

3. The filter according to claim 2, wherein a portion of said fibers is grafted with epoxypropylammonium chloride or epoxypropyltrimethylammonium iodide and the derivatives thereof.

4. The filter according to any of the preceding claims, wherein at least a portion of said fibers is grafted with monomers chosen in the group comprising acrylic acid, alkyl acrylate monomers including 4, 5 or 6 carbon atoms, and acrylonitrile.

5. The filter according to any of the preceding claims, wherein at least a portion of said fibers is grafted with amino derivatives.

6. The filter according to any of the preceding claims, wherein at least a portion of said fibers is grafted with compounds chosen in the group comprising the monomers having double bonds, isoprene, butadiene-styrene and polydiene copolymers.

7. The filter according to any of the preceding claims, wherein at least a portion of said fibers is grafted with silanes or fluorinated monomers.

8. The filter according to any of the preceding claims, wherein for each fiber grafted, the grafting rate is higher or equal to 20 %.